# EUROPEAN PATENT APPLICATION

(11) **EP 4 115 797 A1**
(43) Date of publication of application: **11.01.2023**
(21) Application number: 22183106.8
(22) Date of filing: 05.07.2022
(51) Int. Cl.: A61B 5/00, G06T 7/00, G16H 50/20

(54) **IMAGE CAPTURE SYSTEMS AND METHODS FOR IDENTIFYING ABNORMALITIES USING MULTISPECTRAL IMAGING**

(30) Priority: 06.07.2021 US 202163218551 P
(71) Applicant: Welch Allyn, INC., Skaneateles Falls, NY 13153-0220 (US)
(72) Inventor: LANE, John, Skaneateles Falls, 13153-0220 (US); WHITAKER, Tyson B., Skaneateles Falls, 13153-0220 (US); KINSLEY, Matthew J., Skaneateles Falls, 13153-0220 (US); ROBERTS, Chris R., Skaneateles Falls, 13153-0220 (US); KELLNER, David L., Skaneateles Falls, 13153-0220 (US)
(74) Representative: Findlay, Alice Rosemary

(57) **Abstract**

A method for identifying a skin abnormality including using an imaging device, the imaging device including a lighting member for directing light toward a target surface, the lighting member including a plurality of lighting elements, and a filter member positioned between the lighting member and the target surface, the filter member including a plurality of filter elements, capturing a plurality of images of the target surface, each of the plurality of images captured when illuminating a different one or more of the plurality of lighting elements, compiling the plurality of images into a data package, transmitting the data package to a server for processing the data package, and determining, at the server, a presence of an abnormality.

## Description

The present specification generally relates to identifying skin abnormalities and, more specifically, to image capture systems and methods for identifying skin abnormalities.

Digital imaging devices such as, for example, dermatoscopes, may be used to capture an image of a target area of a subject's skin for purposes of identifying an abnormality. The digital images captured by these devices may be analyzed, such as by a technician, by visually inspecting the digital images to identify the presence of a skin abnormality. However, manual review of each captured image may be time consuming and result in errors such as failure to identify an abnormality and/or misdiagnoses. Additionally, the digital images captured by these digital imaging devices are not designed to capture images of the subject's skin using different image capture constraints. Accordingly, certain abnormalities that may be more distinguishable with different image capture constraints may not be captured by the digital imaging devices that utilize standard or default image capture constraints.

In one aspect, a method for identifying a skin abnormality includes using an imaging device, the imaging device including a lighting member for directing light toward a target surface, the lighting member including a plurality of lighting elements, and a filter member positioned between the lighting member and the target surface, the filter member including a plurality of filter elements, capturing a plurality of images of the target surface, each of the plurality of images captured when illuminating a different one or more of the plurality of lighting elements, compiling the plurality of images into a data package, transmitting the data package to a server for processing the data package, and determining, at the server, the presence of an abnormality.

In another aspect, an abnormality detection system includes an imaging device including a lighting member for directing light toward a target surface, the lighting member including a plurality of lighting elements, and a filter member positioned between the lighting member and the target surface, the filter member including a plurality of filter elements, and a controller configured to capturing a plurality of images of the target surface, each of the plurality of images captured when illuminating a different one or more of the plurality of lighting elements compile the plurality of images into a data package, and transmit the data package to a server for processing the data package, and a server communicatively coupled to the imaging device, the server including a controller configured to receive the data package including the plurality of images captured by the imaging device, and analyze the plurality of images of the data package to detect the presence of a skin abnormality.

In yet another aspect, a dermatoscope includes a lighting member including a plurality of lighting elements, a filter member including a plurality of filter elements for filtering different frequencies of light emitted by the plurality of lighting elements, and a controller configured to capture a plurality of images of a target surface using different combinations of the plurality of lighting elements and the plurality of filter elements by selectively illuminating a different one or more of the plurality of lighting elements for each of the plurality of images, and compile the plurality of images into a data package, and transmit the data package to a server for processing the data package to identify a skin abnormality.

In yet another aspect, a method of manufacturing an imaging device includes using a housing including a first housing portion and a second housing portion defining an interior, a first aperture formed in the first housing portion and a second aperture formed in the second housing portion, positioning an eye piece within the first aperture of the first housing portion, mounting a lighting member within the interior of the housing between the first aperture and the second aperture, the lighting member including a plurality of lighting elements arranged in a spaced apart manner along an outer perimeter of the lighting member; rotatably mounting a filter member within the interior of the housing between the lighting member and the second aperture, the filter member including a plurality of filter elements arranged in a spaced apart manner along an outer perimeter of the filter member, and securing the first housing portion to the second housing portion.

The invention will now be further described by way of example with reference to the accompanying drawings, in which:
FIG. 1 schematically depicts a perspective view of an illustrative imaging device, according to one or more embodiments shown and described herein;
FIG. 2 schematically depicts another perspective view of the imaging device of FIG. 1, according to one or more embodiments shown and described herein;
FIG. 3 schematically depicts an exploded view of the imaging device of FIG. 1, according to one or more embodiments shown and described herein;
FIG. 4 schematically depicts a cross-sectional view of the imaging device of FIG. 1, according to one or more embodiments shown and described herein;
FIG. 5 schematically depicts a lighting member of the imaging device of FIG. 1, according to one or more embodiments shown and described herein;
FIG. 6A schematically depicts a filter member engaging with an adjustment member of the imaging device of FIG. 1, according to one or more embodiments shown and described herein;
FIG. 6B schematically depicts another embodiment of a filter member of the imaging device of FIG. 1, according to one or more embodiments shown and described herein;
FIG. 7 schematically depicts an imaging system including the imaging device of FIG. 1, according to one or more embodiments shown and described herein;
FIG. 8 schematically depicts components of the imaging system of FIG. 7, according to one or more embodiments shown and described herein;
FIG. 9 schematically depicts a controller of the imaging device of FIG. 1, according to one or more embodiments shown and described herein;
FIG. 10 schematically depicts a controller of a server of the imaging system of FIG. 7, according to one or more embodiments shown and described herein; and
FIG. 11 schematically depicts a method for capturing an image using the imaging device of FIG. 1 and the imaging system of FIG. 7 to identify an abnormality, according to one or more embodiments shown and described herein.

Embodiments described herein are directed to abnormality detection systems and methods for identifying a skin abnormality using a multispectral imaging device. The methods described herein include capturing a plurality of images of a target surface with an imaging device that includes a plurality of lighting elements and a plurality of filter elements that are rotatable in front of the plurality of lighting elements to provide a plurality of different combinations of lighting elements and filter elements. The captured images are compiled into a data package, which is transmitted to a server for processing and determining the presence of an abnormality. Various embodiments of the abnormality detection systems and methods and the operation of the abnormality detection systems are described in more detail herein. Whenever possible, the same reference numerals will be used throughout the drawings to refer to the same or like parts.

Unless otherwise expressly stated, it is in no way intended that any method set forth herein be construed as requiring that its steps be performed in a specific order, nor that with any apparatus specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred, in any respect. This holds for any possible non-express basis for interpretation, including: matters of logic with respect to arrangement of steps, operational flow, order of components, or orientation of components; plain meaning derived from grammatical organization or punctuation, and; the number or type of embodiments described in the specification.

As used herein, the singular forms "a," "an" and "the" include plural referents unless the context clearly dictates otherwise. Thus, for example, reference to "a" component includes aspects having two or more such components, unless the context clearly indicates otherwise.

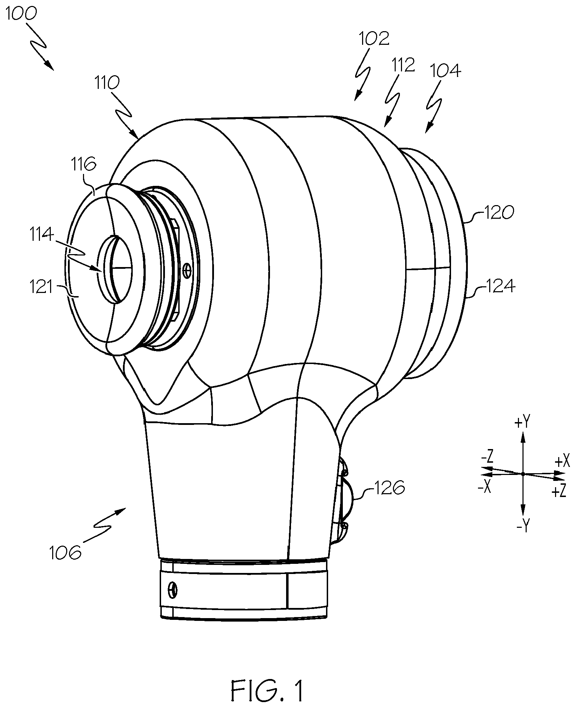

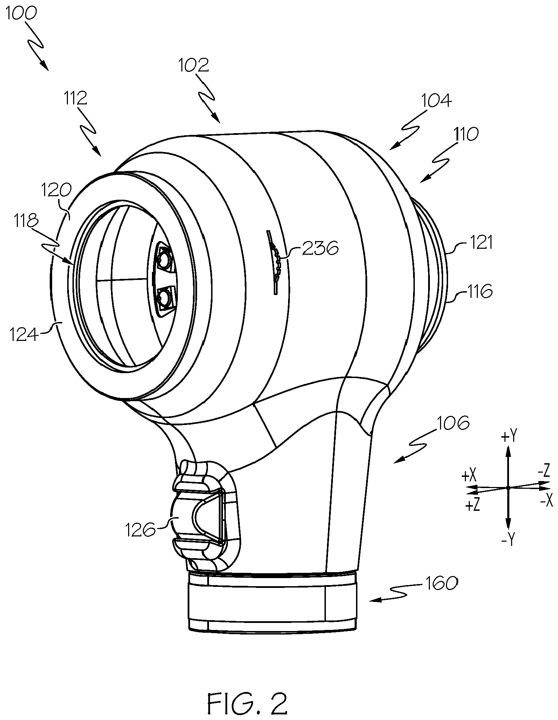

Referring now to FIGS. 1 and 2, an illustrative imaging device 100 is illustrated according to one or more embodiments described herein. The imaging device 100 is depicted as a dermatoscope utilized for examining surfaces of a subject's skin such that an operator may be able to identify abnormalities on the subject's skin. As discussed herein, the abnormalities may be visually identified by the operator, such as by manual digital marking or annotation, or processed using one or more combinations of lighting elements and filter elements to highlight certain characteristics of the surface of the subject's skin, which are processed for identifying the abnormalities. The marking or annotating may indicate a size, such as a diameter, or a shape of a target lesion in the target area of the subject. It should be appreciated that the methods disclosed herein may be utilized with other digital imaging devices that may be capable of alternating between lighting conditions and/or filtering conditions to identify an abnormality on the surface of the subject's skin.

The imaging device 100 may generally include a housing 102 having a body portion 104 for housing internal components, as discussed herein, and a grip portion 106 extending from the body portion 104. In embodiments, the housing 102 may be a one-piece, unitary member. In other embodiments, as shown herein, the housing 102 includes a first housing portion 110 and a second housing portion 112 that are removably joined to one another in any suitable manner. For example, the first housing portion 110 and the second housing portion 112 may be joined using fasteners, clips, adhesives, and/or the like. As described in more detail herein, the first housing portion 110 and the second housing portion 112 may include a mating latch/hook arrangement for securing the first housing portion 110 to the second housing portion 112.

The grip portion 106 is a hollow member in which internal components may be provided. For example, the grip portion 106 may house a power supply, e.g., a battery. Although not shown, the grip portion 106 may also include various electrical or mechanical connectors for connecting the housing 102 to another component or device. For example, the grip portion 106 may include electrical connectors for providing a wired connection with an electric device, such as a mobile device or other computing device. The electrical connectors may also be suitable for wired or wireless recharging of a power supply of the imaging device 100, such as a battery provided within the housing 102 and in electrical communication with the electrical connectors of the grip portion 106. Any suitable mechanical connectors, such as clips, buckles, and/or the like may be provided on the grip portion 106 for removably attaching the housing 102 of the imaging device 100 to another device, component, equipment, and/or surface. For example, the imaging device 100 may be secured to a freestanding floor unit at the grip portion 106. In instances in which the freestanding floor unit is a wheeled piece of equipment, the imaging device 100 may be easily transported by moving the freestanding floor unit to a particular location. In embodiments, the grip portion 106 is shaped so as to be handheld. As used herein, "handheld" refers to the grip portion 106 being shaped such that the grip portion 106 may be easily and comfortably held within the hand of an operator while directing the body portion 104 at the target surface to be captured. More particularly, the grip portion 106 may include individual grooves formed in a surface or side thereof for receiving individual fingers of the operator to accommodate the operator gripping the grip portion 106 of the imaging device 100. The length of the grip portion 106 extending from the body portion 104 may long enough such that the imaging device 100 may be securely gripped by the operator when in use. The grip portion 106 may also be formed from or include a deformable, rubberized, and/or high friction outer coating material to improve comfort and/or avoid slipping when gripped by an operator.

A first aperture 114 is formed at a first side 116 of the housing 102 (e.g., at the first housing portion 110), and a second aperture 118 is formed at a second side 120 of the housing 102 opposite the first side 116 (e.g., at the second housing portion 112). Reference to the first side 116 and the second side 120 of the housing 102 extend along the X-axis. An eyepiece cushion 121 is provided at the first side 116 of the housing 102 concentric with and surrounding the first aperture 114 and a reticle sleeve 124 is provided at the second side 120 of the housing 102 concentric with and surrounding the second aperture 118. The eyepiece cushion 121 may be formed from a deformable material such as, for example, rubber, silicone, or the like to cushion contact by the operator against the housing 102 as the operator brings the imaging device 100 up to his or her eye and looks through the first aperture 114. The reticle sleeve 124 may be a rigid member for securing a reticle 194, discussed in more detail herein, in position against the second aperture 118 of the housing 102. A switch 126 is provided on the grip portion 106 of the housing 102. The switch 126 is generally a user interface component that is shaped, sized, positioned, and/or configured so that the operator may easily activate the imaging device 100 with his or her finger while holding the imaging device 100. The switch 126 may be formed such that the switch 126 is movable such as, for example, via a depressing motion into the grip portion 106 of the housing 102, via a sliding motion in a lateral direction across the grip portion 106, or the like.

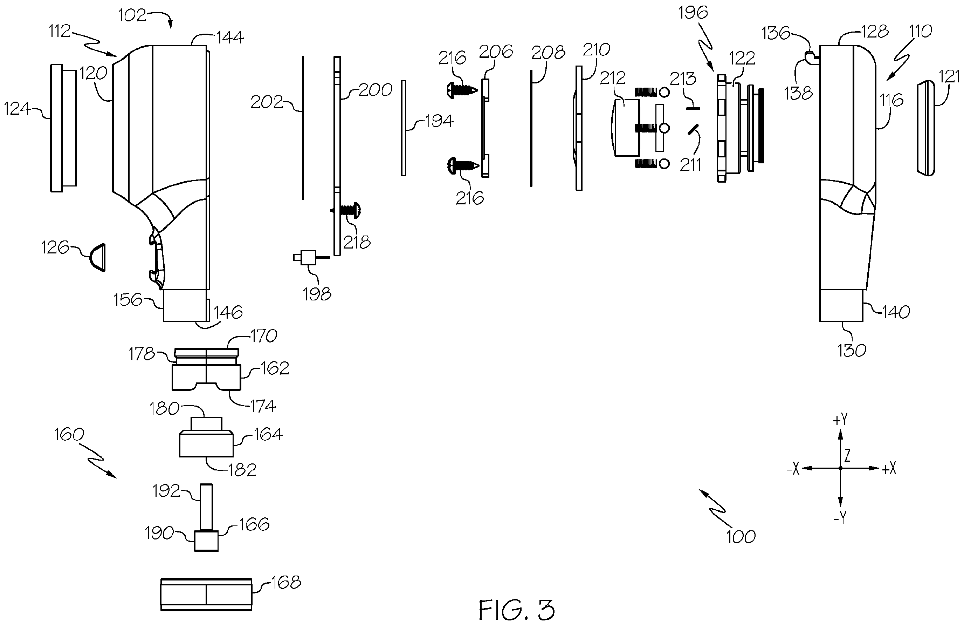

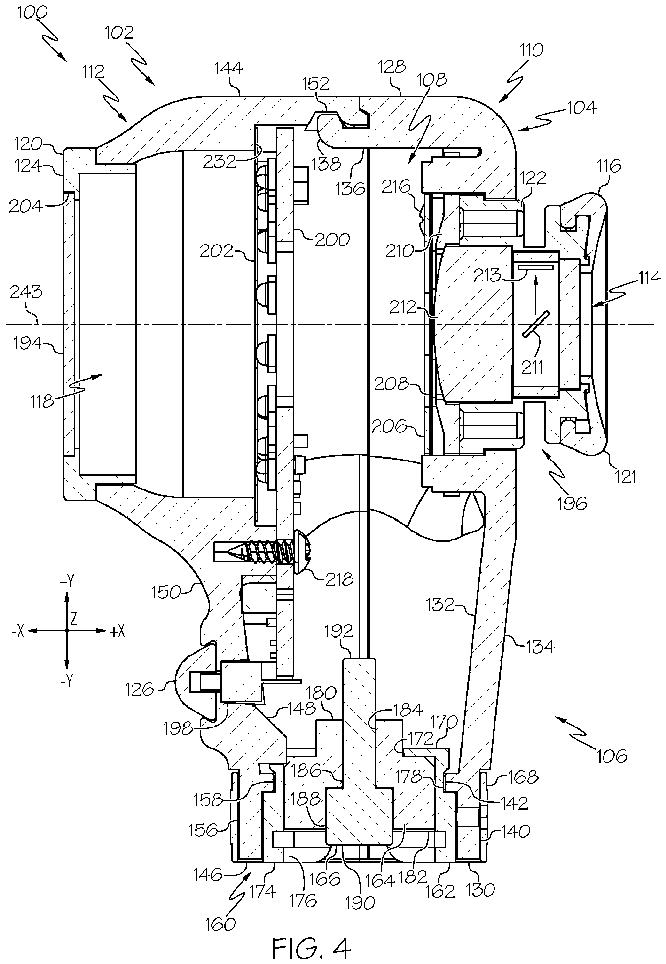

Referring now to FIGS. 3 and 4, the various internal components of the imaging device 100 are illustrated. The body portion 104 and the grip portion 106 define an interior 108 (FIG. 4) of the housing 102 in which various internal components are housed. More particularly, the first housing portion 110 has an upper end 128 at a top of the body portion 104 of the housing 102, a lower end 130 at a bottom of the grip portion 106 housing 102 opposite the upper end 128, an inner surface 132 facing the interior 108 of the housing 102, and an outer surface 134 opposite the inner surface 132. As used herein the top of the body portion 104 and the bottom of the grip portion 106 extends along the +/- Y direction depicted in the coordinate axes. In embodiments, the first housing portion 110 includes a hook 136 extending from the inner surface 132 at the upper end 128 thereof and corresponding to a receptacle 152 formed in the second housing portion 112 and discussed herein that receives the hook 136. However, it should be appreciated that other mechanisms may be provided for joining the first housing portion 110 to the second housing portion 112, such as one or more fasteners, clips, magnets, and the like. The hook 136 has a bend 138 formed therein such that the hook 136 extends toward the outer surface 134 of the first housing portion 110 and opposite the lower end 130. The lower end 130 of the first housing portion 110 is hollow and curved along the outer surface 134 of the grip portion 106, thereby forming a C-shape with an opening of the C-shape facing the opposite second housing portion 112. A stepped portion 140 (FIG. 4) is formed in the outer surface 134 thereof for receiving a trim ring 168, discussed in more detail herein. A flange 142 (FIG. 4) extends along the inner surface 132 of the first housing portion 110 proximate the lower end 130 thereof and perpendicular to the inner surface 132 of the first housing portion 110.

The second housing portion 112 also has an upper end 144 at a top of the body portion 104 of the housing 102, a lower end 146 at a bottom of the grip portion 106 of the housing 102 opposite the upper end 128, an inner surface 148 facing the interior 108 of the housing 102, and an outer surface 150 opposite the inner surface 148. In embodiments, the second housing portion 112 includes a receptacle 152 formed in the inner surface 148 at the upper end 128 configured to receive the hook 136 of the first housing portion 110, as shown in FIG. 4, such that the upper end 128 of the first housing portion 110 may be removably secured to the upper end 144 of the second housing portion 112. In embodiments, the second housing portion 112 may include the hook 136 and the first housing portion 110 may include the receptacle 152. Similar to the first housing portion 110, the lower end 146 of the second housing portion 112 is hollow and curved along the outer surface 150 of the grip portion 106, thereby forming a C-shape with an opening of the C-shape facing the opposite first housing portion 110. A stepped portion 156 (FIG. 4) formed in the outer surface 150 thereof for receiving the trim ring 168, discussed in more detail herein. A flange 158 (FIG. 4) extends along the inner surface 148 of the second housing portion 112 proximate the lower end 146 thereof and perpendicular to the inner surface 148 of the second housing portion 112.

Referring still to FIGS. 3 and 4, the imaging device 100 includes a grip securing mechanism 160 for securing the lower end 130 of the first housing portion 110 to the lower end 146 of the second housing portion 112. The grip securing mechanism 160 includes a stud contact 162, an insulator 164, a contact pin 166, and a trim ring 168. Specifically, the stud contact 162 has an upper end 170 defining an upper opening 172 formed therein, and an opposite lower end 174 defining a lower opening 176 formed therein. A diameter of the lower opening 176 is greater than a diameter of the upper opening 172. Although an interior wall extending between the upper opening 172 and the lower opening 176 is shown as having a step formed therein, the interior wall may have a constant slope or curve extending therebetween. A recess 178 is formed along an exterior surface of the stud contact 162 closer to the upper end 170 than to the lower end 174. As discussed in more detail herein, the recess 178 is formed to receive the flanges 142, 158 of the first housing portion 110 and the second housing portion 112.

The insulator 164 has an upper portion 180 having an upper outer diameter and an opposite lower portion 182 having a lower outer diameter greater than the upper outer diameter. In embodiments, the upper outer diameter of the insulator 164 is equal to or less than the upper diameter of the upper opening 172 formed in the upper end 170 of the stud contact 162 so that the insulator 164 is able to partially extend through the upper opening 172 formed in the stud contact 162. Additionally, the lower outer diameter of the insulator 164 is equal to or less than the lower diameter of the lower opening 176 formed in the lower end 174 of the stud contact 162 so that the insulator is contained within the stud contact 162. As such, it can be appreciated that the insulator 164 is positionable within an interior of the stud contact 162. The insulator 164 includes a bore 184 extending from the lower portion 182 to the upper portion 180. The bore 184 has a first portion 188 proximate the lower portion 182 having a first diameter and a second portion 186 proximate the upper portion 180 having a second diameter less than the first diameter. The insulator 164 may be formed from any suitable insulating material. In embodiments, the insulator 164 is formed from rubber and, more particularly, silicone rubber. The insulator 164 may be suitable for insulating the contact pin 166 from the stud contact 162 and the housing 102 of the imaging device 100.

The contact pin 166 includes a head 190 and a shaft 192 extending from the head 190. The contact pin 166 is positionable within the bore 184 of the insulator 164 such that the head 190 of the contact pin 166 is received within the first portion 188 of the bore 184 and the shaft 192 is received within the second portion 186 of the bore 184. In embodiments, the contact pin 166 includes an elastomer coating allowing the contact pin 166 to be press fit into the insulator 164. In other embodiments, the insulator 164 includes an elastomer such that the insulator 164 may be press fit within the stud contact 162. As discussed herein, the grip portion 106 may house a power supply, e.g., a battery, and include various electrical or mechanical connectors for connecting the housing 102 to another component or device. In embodiments, the contact pin 166 functions as an electrical connector for electrically coupling the imaging device 100 to an external power source for charging a power supply of the imaging device 100 or otherwise providing power to the imaging device 100. Accordingly, the head 190 of the contact pin 166 may communicate with a power source via wired communication or wireless communication such as, for example, inductive charging. An end of the shaft 192 of the contact pin 166 opposite the head 190 transfers power from the power source to the one or more components within the imaging device 100 such as the power supply, which then delivers power to the other components. In embodiments, the shaft 192 engages the power supply provided within the grip portion 106 to couple the power supply to the power source external of the imaging device 100.

As shown in FIG. 4, when the first housing portion 110 is engaged with the second housing portion 112, the stud contact 162 is positioned between the lower end 130 of the first housing portion 110 and the lower end 146 of the second housing portion 112 such that the flange 142 of the first housing portion 110 and the flange 158 of the second housing portion 112 are received within the recess 178 in the stud contact 162. The insulator 164 and the contact pin 166 are portioned within an interior of the stud contact 162. To finally secure the first housing portion 110 and the second housing portion 112, the trim ring 168 is positioned around the lower end 130 of the first housing portion 110 and the lower end 146 of the second housing portion 112, particularly within the stepped portions 140, 156 formed in the outer surfaces 134, 150 thereof.

Referring again to FIGS. 3 and 4, the internal components of the imaging device 100 within the interior 108 defined by the housing 102 are illustrated. In embodiments, the imaging device 100 generally includes a reticle 194, a lens assembly 196, a switch receiver 198, a lighting member 200, and a filter member 202. It should be appreciated that the imaging device 100 may include additional or fewer components other than those depicted in FIGS. 3-4 without departing from the scope of the present disclosure.

The reticle 194 is positioned at the second aperture 118 of the housing 102. The reticle 194 is nested within a groove 204 formed in the second housing portion 112 at the second aperture 118 such that the groove 204 retains the reticle 194 therein. In embodiments, the reticle 194 may include a plurality of fine lines or fibers extending across a viewing surface of the reticle 194 that serve as a reference for measuring objects viewed by the imaging device 100. Based on a known distance between the fine lines or fibers, a distance measurement of the captured target area may be determined. More particularly, the fine lines or fibers may be utilized as a reference scale to determine a distance measurement of the captured target area. Accordingly, after capturing an image of the target area, a distance across the entire target area may be compared to a distance between two or more of the fine lines or fibers to determine a diameter and other measurements of the target area, such as a specific lesion in the target area.

The lens assembly 196 may be any well-known digital imaging mechanism, either commercially available or to-be-developed, to capture images of an object viewed by the imaging device 100. Such imaging mechanisms include those provided in digital scopes such as dermatoscopes, otoscopes, and the like. In embodiments, the lens assembly 196 includes a retainer 206, a polarizer 208, a frame 210, a lens 212, an eyepiece 122, and an image sensor 213. The retainer 206 secures each of the components of the lens assembly 196 within the first housing portion 110 proximate the first aperture 114. More particularly, one or more fasteners 216 extend through the retainer 206 and engage the first housing portion 110, thereby sandwiching the components of the lens assembly 196 between the retainer 206 and the first side 116 of the housing 102 and within the body portion 104 of the housing 102.

The image sensor 213 may be any suitable imaging device, for example, CCD, CMOS, and the like, positioned such that light passing through the second aperture 118 is captured by the image sensor 213 and may also be substantially simultaneously viewable by a user through the eyepiece 122. As such, a light path between the second aperture 118 and the image sensor 213 and/or the first aperture 114 may be defined by any combination of optical components (e.g., mirrors, prisms, lenses, and/or the like) for focusing light, redirecting light, and/or the like. For example, the image sensor 213 and/or the first aperture 114 may be coupled to or adjacent to one or more optical components for receiving light passing through the second aperture 118. As shown, a light reflecting element 211 may be arranged within the lens assembly 196, for example, within the eyepiece 122, at an oblique angle relative to an axis 243 of the imaging device 100 along which light entering the second aperture 118 travels, as described in more detail herein. The light reflecting element 211 may be a mirror, which may include one or more rotating mirrors, a MEMS mirror, and/or the like. The light reflecting element 211 may also be a prism, which may include a pentaprism and/or the like. Accordingly, the light reflecting element 211 reflects light entering the housing 102 through the second aperture 118, the lighting member 200, and the filter member 202 along the axis 243 to the image sensor 213 so that the image may be captured by the image sensor 213 so that the image can be captured. In embodiments where the light reflecting element 211 is a movable mirror or the like, the movement of the light reflecting element 211 may be such that the light from the second aperture 118 results in an image being viewed by the user and an image captured by the image sensor 213 substantially simultaneously (e.g., in rapid succession such that the difference is imperceptible to a human viewer). In embodiments, actuation of the switch 126 may result in the light reflecting element 211 selectively reflecting the image to the image sensor 213 and/or allowing the image to pass through, or avoid, the light reflecting element 211 such that the image may be viewed by the user. This may be permitted by adjusting a position of the light reflecting element 211 between the position shown in FIG. 4 in which the image is reflected to the image sensor213 and a flipped up or rotated position in which the light reflecting element 211 is moved out of the path of the axis 243 to permit the image to pass to the user unobstructed and/or adjusting a transparency of the light reflecting element 211 to allow light to either pass through or be reflected. It should be appreciated that the light reflecting element 211 may include one or more motors, actuators, or the like for positioning the light reflecting element 211 in the manner discussed herein and/or one or more electrodes, compositions, and/or the like to allow for transparency switching. The image sensor 213 is in communication with a controller 400 of the imaging device 100, as discussed in more detail herein, and operated to capture images viewed through the lens assembly 196. Although the image sensor 213 is illustrated as being positioned within the imaging device 100 at the eyepiece 122, it should be appreciated that the image sensor 213 may be positioned exterior of the imaging device 100. For example, the image sensor 213 may be an external image capture device or a component of a larger external device that is attached to the first housing portion 110, such as at the first side 116 of the housing 102. In this embodiment, the image sensor 213 may be included in a mobile device and positioned such that a field of view of the image sensor 213 of the mobile device captures an image of the reflected light. The mobile device including the image sensor 213 may be removably mounted to the housing 102 in any suitable manner such as, for example, one or more fasteners, clips, magnets, and the like.

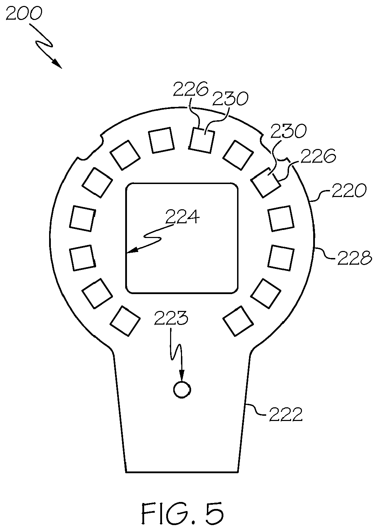

The lighting member 200 is positioned between the lens assembly 196 and the filter member 202 and is secured to the second housing portion 112 by one or more fasteners 218. Referring now to FIG. 5, the lighting member 200 includes a faceplate 220 and a body 222 extending from the faceplate 220. The body 222 defines an opening 223 through which the fastener 218 (FIG. 4) extends to secure the lighting member 200 to the second housing portion 112. In embodiments, the faceplate 220 of the lighting member 200 has an opening 224 and a plurality of holes 226 formed along an outer perimeter 228 of the faceplate 220 surrounding the opening 224. As shown, the opening 224 may be centrally located on the faceplate 220. However, the opening 224 may be positioned in some other location that is not central on the faceplate 220. Additionally, as shown, the opening 224 has a square shape with rounded corners. However, the opening 224 may have any other suitable shape to permit light to pass through the lighting member 200. A lighting element 230 is positioned within each one of the holes 226 and in electrical communication with the controller 400 of the imaging device 100. In embodiments, the controller 400 may be provided on a surface of the body 222 of the lighting member 200. In other embodiments, the lighting elements 230 may be directly mounted on the faceplate 220, thereby eliminating the need for the holes 226 to be formed in the lighting member 200 and the lighting elements 230 positioned therein.

The lighting member 200 includes a plurality of lighting elements 230 arranged in a spaced apart manner around the faceplate 220. In embodiments, the lighting elements 230 may be light emitting diodes (LEDs) or any other light source capable of emitting light to illuminate a surface of the skin of the subject at which the imaging device 100 is directed. In embodiments, the lighting member 200 includes a plurality of lighting elements 230 that emit light at a variety of different frequencies or wavelengths. For example, a first one or more of the lighting elements 230 may be an infrared lighting element for emitting infrared light and a second one or more of the lighting elements 230 may be an ultraviolet lighting element for emitting ultraviolet light. Accordingly, the lighting member 200 may include a plurality of each of the different lighting elements 230. Similar lighting elements 230, e.g., ultraviolet lighting elements, may be arranged adjacent to one another or, alternatively, separated by other lighting elements 230. It should be appreciated that the lighting elements 230 are independently operated by the controller 400 to illuminate the surface of the skin of the subject based on the particular needs of the imaging device 100 and the particular abnormality to be imaged. Additional or alternative lighting elements 230 emitting different wavelengths of light may be provided other than those discussed herein. In embodiments, the lighting elements 230 may be provided on a single lighting strip or elongated lighting element extending along the lighting member 200 rather than including a plurality of individual lighting elements 230. In embodiments, the lighting member 200 may be a printed circuit board including the plurality of lighting elements 230 coupled thereto. In embodiments, the lighting elements 230 are tunable such that each lighting element 230 is capable of emitting light at more than one wavelength. This allows each of the lighting elements 230 to emit light of the same wavelength or a different wavelength. The lighting elements 230 may also be tunable such that each lighting element 230 is capable of emitting a brightness having varying intensity. The brightness of each lighting element 230 may be manually adjusted or automatically adjusted depending on the conditions in which the imaging device 100 is operating, for example, detecting lighting conditions surrounding the imaging device 100 and/or detecting a distance of the imaging device 100 from the subject's skin. In addition, the lighting elements 230 may be fixed to the lighting member 200 such that each of the lighting elements 230 are specifically aimed at a particular orientation or angle. Illumination of different lighting elements 230 at different angles may result in a particular illumination of the target area, thereby ensuring a particular size of the target area is illuminated when the imaging device 100 is positioned at a certain distance from the subject's skin.

Referring again to FIGS. 3 and 4, the filter member 202 is positioned between the lighting member 200 and the reticle 194. More particularly, the filter member 202 is secured between the lighting member 200 and a ledge 232 of the second housing portion 112 by the one or more fasteners 218 securing the filter member 202. As discussed in more detail herein, the filter member 202 is rotatable within the housing 102 about an axis 243 (FIG. 4) extending through the opening 224 of the lighting member 200 in embodiments.

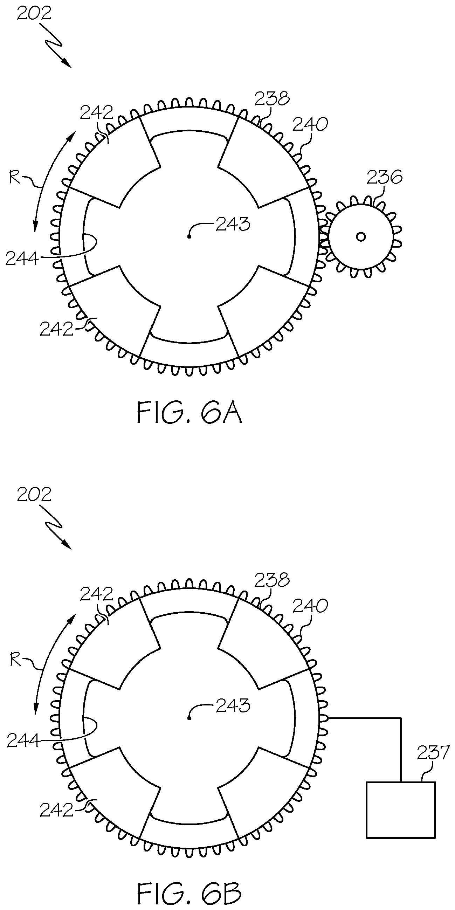

Referring now to FIG. 6A, the filter member 202 is illustrated. In embodiments, the filter member 202 includes a plurality of filter elements 242 that alter the wavelength of the light passing through the filter elements 242. For example, a first one or more of the filter elements 242 may be a white filter for filtering white light, a second one or more of the filter elements 242 may be a green filter for filtering green light, and a third one or more of the filter elements 242 may be a red filter for filtering red light. One or more of the filter elements 242 may also be a bandpass filter, a polarizing filter, and/or the like. Thus, by rotating the filter member 202 amount the lighting member 200, various combinations of lighting elements 230 and filter elements 242 may be provided for highlighting certain characteristics of the subject's skin. Additionally, the lens assembly 196 may capture a plurality of images as the filter member 202 is rotated and individual lighting elements 230 of the lighting member 200 are illuminated. As described in more detail herein, the capturing of a plurality of images provides a multispectral imaging technique in which the plurality of captured images may be layered on top of one another and processed simultaneously.

As shown in FIG. 6A, the filter elements 242 are spaced apart from one another to provide a gap 244 between adjacent filter elements 242. Thus, as described in more detail herein, a lighting element 230 may be positioned behind a particular filter element 242 to create a specific combination of filtered light such as, for example, infrared light emitted from a lighting element 230 being filtered by a white filter. Alternatively, the illuminated lighting element 230 may be positioned between two filter elements 242 such that the light emitted from the lighting element 230 is not filtered by any of the filter elements 242. In embodiments, the filter elements 242 may be positioned and/or arranged such that one or more of the filter elements 242 are configured to filter the outgoing light emitted from an illuminated lighting element 230 and a different one or more filter elements 242 are configured for further filtering of the incoming light received at the imaging device 100. For example, the one or more filter elements 242 that filter the outgoing light may be positioned in front of the illuminated lighting element 230 and the different one or more filter elements 242 that filter the incoming light may be positioned in front of the imaging device 100, rather than the illuminated lighting element 230. Alternatively, some of the filter elements 242 may be one-way filters such that light is only filtered when passing in one direction through the filter elements 242. Thus, certain filter elements 242 only filter outgoing light while other filter elements 242 only filter incoming light.

In embodiments, an adjustment member 236 is accessible from an exterior of the housing 102, which engages the filter member 202, particularly an outer perimeter 238 of the filter member 202. Accordingly, operation of the adjustment member 236 from an exterior of the housing 102 causes rotation of the filter member 202. In embodiments, the adjustment member 236 may be a thumb wheel that engages a plurality of teeth 240 formed along the outer perimeter 238 of the filter member 202. Accordingly, rotation of the thumb wheel in one direction results in rotation of the filter member 202 in an opposite direction. In other embodiments, the adjustment member 236 may be controlled by the controller 400 such that the filter member 202 may be rotated upon a signal transmitted from the controller 400. In embodiments in which the filter member 202 is rotated manually, the plurality of teeth 240 formed on the outer perimeter 238 of the filter member 202 engage the adjustment member 236, which is illustrated as a thumb wheel. The adjustment member 236 is rotatably mounted to the housing 102 and accessible from an exterior of the housing 102. Thus, rotation of the adjustment member 236 engages the filter member 202 and causes the filter member 202 to rotate about an axis 243 extending between the first side 116 and the second side 120 of the housing 102. Accordingly, different filter elements 242 of the filter member 202 may be aligned with different lighting elements 230 of the lighting member 200 to create and capture different combinations of illuminated and filtered images.

In some embodiments, the filter member 202 is manually rotatable. That is, a portion or portions of the filter member 202 may be accessible from an exterior of the housing 102 and such portion or portions may be directly rotatable by a user rather than being indirectly rotatable via the adjustment member 236 as described herein. In other embodiments, a lever mechanism (not shown) may be provided and operably coupled to the filter member 202. The lever mechanism may be spring-loaded or otherwise coupled to a biasing assembly such that operation of the lever mechanism in a first direction, such as by lifting, rotating, and/or the like, results in a fixed amount of rotation of the filter member 202 in the first direction. Upon release of the lever mechanism, the lever mechanism is biased back toward an initial unbiased position to be operated again, thereby further rotating the filter member 202 in the first direction. Each rotation of the lever mechanism results in incremental rotation of the filter member 202 to position a different one or more of the filter elements 242 in position relative to the lighting elements 230. The fixed amount of rotation of the filter member 202 may be defined by a plurality of protrusions formed in the perimeter of the filter member 202 that engage with a notch formed in the housing 102. Thus, with each rotation of the filter member 202, the notch engages a different one of the protrusions with each operation of the lever mechanism. Alternatively, the notch may be formed in the filter member 202 and the protrusions may be formed in the housing 102.

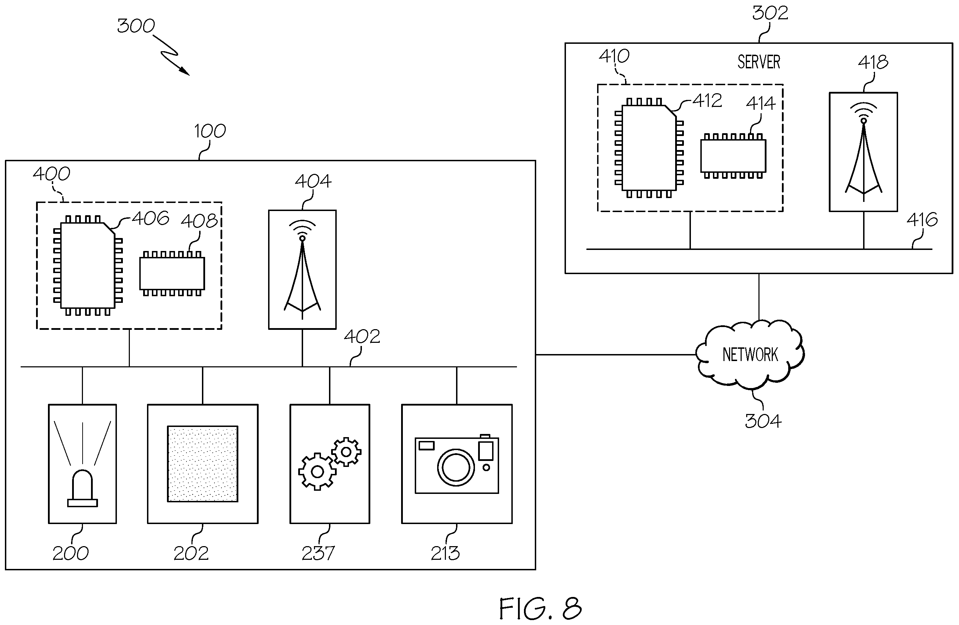

Referring now to FIG. 6B, in embodiments in which the filter member 202 is automatically rotated, the filter member 202 may be rotated using any suitable device such as a motor 237 or actuator driving a rotatable shaft (not shown) coupled to the filter member 202. In embodiments, the motor 237 may be a stepper motor configured to rotate the filter member 202 in one or more steps in a rotational direction R. When the filter member 202 is configured to automatically rotate, the motor 237 may be coupled to a controller that is preprogrammed to cause specific operation of the motor 237 for a particular amount of time or permit a limited amount of rotation with each activation. Thus, each actuation of the motor 237 will incrementally rotate the filter member 202 such that a different filter element 242 is positioned relative to a lighting element 230. It should be appreciated that the controller 400 (FIG. 8) may be preprogrammed, calibrated, or otherwise configured to track movement and/or a position of the filter member 202 to determine at any given time a position of the filter member 202 and, particularly, an orientation of the filter member 202 to determine which filter elements 242 are positioned in front of which lighting elements 230.

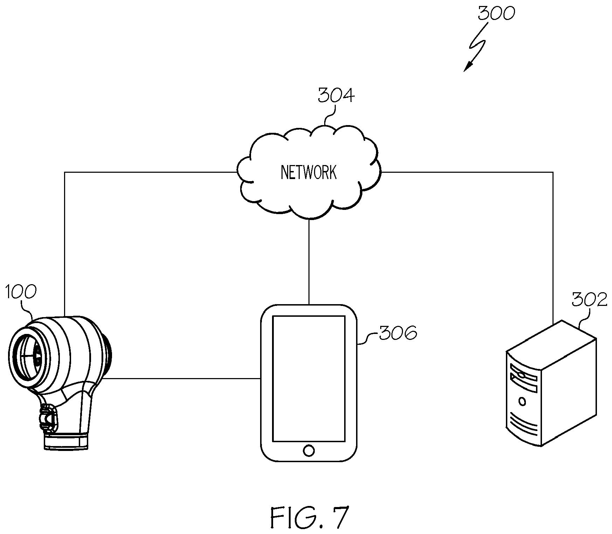

Referring now to FIG. 7, a schematic diagram of an embodiment of an abnormality detection system 300 is depicted. The abnormality detection system 300 includes the imaging device 100, a server 302, and/or a mobile device 306 communicatively coupled to one another via a direct communication link and/or via a network 304. As described in more detail herein, the imaging device 100 is operable to capture an image of the skin of the subject. The images captured by the imaging device 100 may be transmitted to the server 302 via the network 304 for processing to determine whether the irregularity is present. It should be appreciated that, in embodiments, the server 302 may be a local device communicatively coupled to the imaging device 100 via a wired connection rather than the server 302 being a remote device.

In embodiments, the mobile device 306 may receive signals and/or instructions corresponding to particular device parameters that are selected based on the subject and display instructions indicating how the imaging device 100 should be operated to capture images. For example, based on the parameters received at the mobile device 306, the mobile device 306 may instruct the imaging device 100 to utilize a particular combination of lighting elements 230 and/or filter elements 242. In addition, the mobile device 306 may instruct the imaging device 100 to alternate between a combination of lighting elements 230 and/or filter elements 242 to capture a plurality of images. After the captured images are transmitted to the server 302, either by the imaging device 100 or the mobile device 306 via the network 304, the server 302 may process the captured images and determine that additional images are necessary to accurately determine whether an abnormality is present. Accordingly, the server 302 may send instructions to the mobile device 306 via the network 304 as to the specific parameters, e.g., combinations of lighting elements 230 and filter elements 242, that are necessary to capture additional images. The mobile device 306 may display the parameters to the operator such that the imaging device 100 can be adjusted either manually or automatically to position the filter member 202 and illuminate the particular lighting elements 230. Although referred to herein as the mobile device 306, it should be appreciated that the mobile device 306 may be incorporated onto the imaging device 100. For example, the mobile device 306 may include a display screen on the imaging device 100 itself such that the information provided by the mobile device 306 is readily apparent to the operator of the imaging device 100 and eliminates the need for a separate device. In other embodiments, the mobile device 306 may be some other computing device or remote terminal such as, for example, a television or display provided in an operating room or subject's room.

Referring now to FIG. 8, various additional intemal components of the imaging device 100 and internal component of the server 302 are disclosed. The imaging device 100 includes the controller 400, a communication path 402, network interface hardware 404, the lighting member 200, the filter member 202, and the image sensor 213. The various components of the imaging device 100 and the interaction thereof will be described in detail below. It should be noted that some embodiments of the imaging device 100 may include additional components not described herein.

The communication path 402 may be formed from any medium that is capable of transmitting a signal such as, for example, conductive wires, conductive traces, optical waveguides, or the like. Moreover, the communication path 402 may be formed from a combination of mediums capable of transmitting signals. In one embodiment, the communication path 402 includes a combination of conductive traces, conductive wires, connectors, and buses that cooperate to permit the transmission of electrical data signals to components such as processors, memories, sensors, input devices, output devices, and communication devices. Accordingly, the communication path 402 may include a bus, such as for example a LIN bus, and the like. Additionally, it is noted that the term "signal" means a waveform (e.g., electrical, optical, magnetic, mechanical or electromagnetic), such as DC, AC, sinusoidal-wave, triangular-wave, square-wave, vibration, and the like, capable of traveling through a medium. The communication path 402 communicatively couples the various components of the server 302. As used herein, the term "communicatively coupled" means that coupled components are capable of exchanging data signals with one another such as, for example, electrical signals via conductive medium, electromagnetic signals via air, optical signals via optical waveguides, and the like.

As noted above, the imaging device 100 includes the controller 400 including the one or more processors 406 and one or more memory components 408. Each of the one or more processors 406 may be any device capable of executing machine readable instructions (e.g., non-transitory, machine readable instructions). Accordingly, each of the one or more processors 406 may be an integrated circuit, a microchip, a computer, or any other computing device. The one or more processors 406 are communicatively coupled to the other components of the imaging device 100 by the communication path 402. Accordingly, the communication path 402 may communicatively couple any number of processors with one another, and allow the modules coupled to the communication path 402 to operate in a distributed computing environment. Specifically, each of the modules may operate as a node that may send and/or receive data.

Each of the one or more memory components 408 of the imaging device 100 is coupled to the communication path 402 and communicatively coupled to the one or more processors 406. The one or more memory components 408 may include RAM, ROM, flash memories, hard drives, and/or any other hardware or firmware capable of storing machine readable instructions such that the machine readable instructions may be accessed and executed by the one or more processors 406 (e.g., non-transitory memory components). The machine readable instructions may include logic or algorithm(s) written in any programming language of any generation (e.g., 1GL, 2GL, 3GL, 4GL, or 5GL) such as, for example, machine language that may be directly executed by the processor, or assembly language, object-oriented programming (OOP), scripting languages, microcode, and the like, that may be compiled or assembled into machine readable instructions and stored on the one or more memory components 408. In some embodiments, the machine readable instructions may be written in a hardware description language (HDL), such as logic implemented via either a field-programmable gate array (FPGA) configuration or an application-specific integrated circuit (ASIC), or their equivalents. Accordingly, the methods described herein may be implemented in any conventional computer programming language, as pre-programmed hardware elements, or as a combination of hardware and software components.

The lighting member 200 is coupled to the communication path 402 and communicatively coupled to the one or more processors 406. Accordingly, the lighting member 200 may be operated in response to instruction from the one or more processors 406 to illuminate one or more of the lighting elements 230. For example, the lighting member 200 may be instructed to operate or tune a single lighting element 230 or a plurality of lighting elements 230 either simultaneously or sequentially to emit light with the necessary lighting constraints. As the lighting member 200 is operated and the one or more lighting elements 230 are illuminated, the imaging device 100 is instructed to capture an image of the surface of the skin of the subject.

In embodiments in which the filter member 202 may be automatically rotated, the filter member 202, particularly the motor 237 (FIG. 6B), is coupled to the communication path 402 and communicatively coupled to the one or more processors 406. Accordingly, the filter member 202 may be operated in response to instruction from the one or more processors 406 to be rotated if supporting motor, such as the motor 237 or gear members are provided. For example, the filter member 202, particularly the motor 237, may be instructed to operate to rotate the filter member 202 such that a particular filter element 242 is positioned in front of a lighting element 230 of the lighting member 200 to be illuminated.

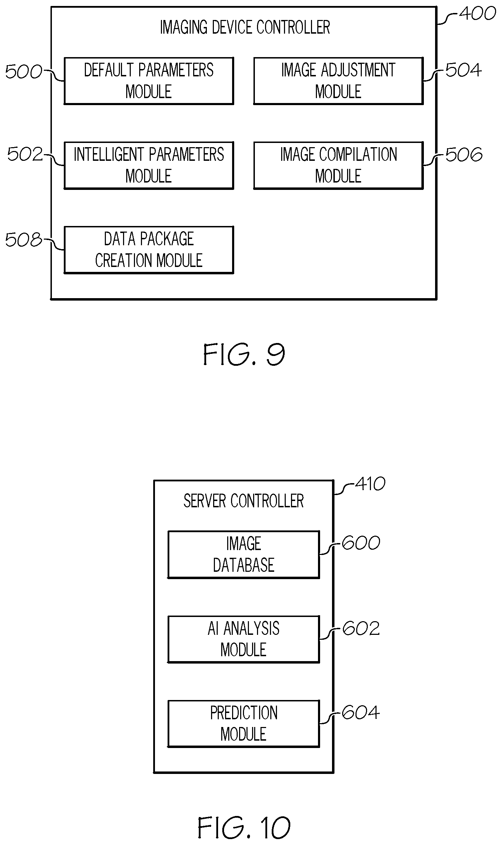

Referring now to FIG. 9, individual modules of the controller 400 of the imaging device 100 is illustrated. Although the modules are discussed herein as being provided in the imaging device 100, it should be understood that corresponding modules carrying out similar functions may alternatively be provided in a separate device, such as the mobile device 306. Accordingly, the controller 400 of the imaging device 100 includes a default parameters module 500, an intelligent parameters module 502, an image adjustment module 504, an image compilation module 506, and a data package creation module 508.

The default parameters module 500 includes programming that provides functionality for providing default parameters for capturing an initial image or plurality of images of the subject. The default parameters include various settings, positions, operation instructions, and the like for the lighting member 200 and/or the filter member 202. Non-limiting examples of the default parameters may be illuminating only one of the lighting elements 230 of the lighting member 200, rotating the filter member 202 to position a particular filter element 242 in front of a particular lighting element 230, or subsequently illuminating a series of lighting elements 230 behind particular filter elements 242 to capture a plurality of images using a variety of different light and filter combinations.

The intelligent parameters module 502 includes programming that provides functionality for adjusting the default parameters set by the default parameters module 500 based on one or more identified characteristics of the subject. For example, the imaging device 100 may identify certain lighting conditions surrounding the subject and, more particularly, the area of the subject's skin to be captured by the imaging device 100, and adjust the default parameters based on these conditions. As another example, the imaging device 100 may identify that the area of the subject's skin to be captured has certain characteristics requiring specific lighting and filter characteristics other than that provided by the default parameters. Thus, the intelligent parameters module 502 updates the default parameters retrieved by the default parameters module 500 to capture one or more images using these updated parameters. In embodiments, the intelligent parameters module 502 is updatable by receiving information from an external device, such as via a machine learning server, or the like. In other embodiments, the intelligent parameters module 502 contains code, such as machine learning code and/or a trained artificial neural network that allows the intelligent parameters module 502 to be updated without receiving any external information.

The image adjustment module 504 includes programming that provides functionality for adjusting the captured images so that the captured images are similarly oriented and sized such that the location of the target area of the subject's skin captured by the imaging device 100 is similarly positioned across each of the captured images. For example, the image adjustment module 504 may crop each captured image to remove extraneous imagery captured of the subject. In addition, the image adjustment module 504 may rotate each captured image so that the target area is oriented in the same direction in each captured image. Specifically, the image adjustment module 504 may identify one or more target points on the target area in each captured image and rotate the captured images so that the one or more target points are in the same position relative to a horizontal axis and a vertical axis in each captured image. In addition, the image adjustment module 504 may adjust certain lighting characteristics in each captured image such as, for example, contrast, brightness, and the like so that the captured images appear to be subjected to the same lighting conditions.

The image compilation module 506 includes programming that provides functionality for compiling the captured images and, in embodiments, processes the captured images to identify whether the imaging device 100 should capture one or more additional images using a different combination of lighting elements 230 and filter elements 242. The image compilation module 506 may determine that additional images are required by identifying key characteristics within the captured images and determining a combination of lighting elements 230 and filter elements 242 that may visually enhance the key characteristics. In response to identifying a combination of lighting elements 230 and filter elements 242 that may enhance the key characteristics, the image compilation module 506 sends instructions to the imaging device 100 to capture one or more additional images based on the determined lighting combinations of lighting elements 230 and filter elements 242. As discussed in more detail herein, the image compilation module 506 may receive previously captured images from either the imaging device 100 or a different source and organizes the captured images chronologically. As such, the captured images, along with those previously captured images, may be viewed to identify progressions of the subject's skin taken over a period of time. In embodiments, the image adjustment module 504 and the image compilation module 506 may be combined into a single module.

The data package creation module 508 includes programming that provides functionality for preparing the captured images to be transmitted to the server 302 for processing. Specifically, the data package creation module 508 will apply a date and time stamp to each captured image. In embodiments, the data package creation module 508 may create a plurality of merged image files with each image file including one or more combinations of a plurality of the captured images being overlaid on top of one another. Overlaying the captured images on top of one another allows for a combination of images to be simultaneously processed by the server 302. This method of processing a plurality of captured images simultaneously is referred to herein as "multispectral imaging" in which a plurality of images of the target area of the subject are captured using different combinations of lighting elements 230 and filter elements 242, and subsequently processing the captured images simultaneously as they are overlaid on top of one another. This technique of multispectral imaging allows for more accurate detection of abnormalities within the target area of the subject and shorter processing times. Alternatively, the captured images including various combinations of lighting elements 230 and filter elements 242 may be separately processed without overlaying the captured images on top of one another. However, this may increase the processing time by requiring each captured image to be processed individually. In embodiments, the image adjustment module 504, the image compilation module 506, and the data package creation module 508 may be combined into a single module.

Referring again to FIG. 8, the server 302 includes a controller 410, including one or more processors 412 and one or more memory components 414, a communication path 416, and network interface hardware 418. The various components of the server 302 and the interaction thereof will be described in detail below. However, it should be noted that, in embodiments, the server 302 may include fewer components than those discussed herein or additional components not discussed herein.

The components of the server 302 may be structurally similar to and have similar functions as the corresponding components of the imaging device 100 (e.g., the controller 400 corresponds to the controller 410, the communication path 402 corresponds to the communication path 416, and the network interface hardware 404 corresponds to the network interface hardware 418).

Referring now to FIG. 10, individual modules of the controller 410 of the server 302 is illustrated. The controller 410 of the server 302 includes an image database 600, an artificial intelligence (Al) analysis module 602, and a prediction module 604.

The image database 600 includes a plurality of test images and diagnoses associated with each test image. The image database 600 may be a remote server hosted by an entity separate from the server 302 (e.g., a cloud-based image database, a government repository, a research facility repository, and/or the like). The test images may be received from one or more publicly available databases or private databases in which the images have been previously captured and processed to identify the presence of an abnormality on a subject's skin. Accordingly, each test image is provided with data including, for example, demographic information of the subject, such as age, gender, and nationality, date and time that the image was captured, and whether or not an abnormality is present, as well as the type of abnormality. It should be appreciated that the image database 600 may be continually updated to receive additional or updated test images and diagnoses from any remote database via the network interface hardware 418 of the server 302. As discussed in more detail herein, the images captured by the imaging device 100 may be compared to the test images within the image database 600 to determine whether an abnormality is present. This comparison may be done manually by a technician or automatically using machine learning and artificial intelligence.

Accordingly, the AI analysis module 602 includes an artificial neural network trained by processing the test images in the image database 600. For example, the artificial neural network is initially trained by providing a number of test data as inputs. The inputs may include the test images of the image database 600, including the data associated with each test image. The artificial neural network includes a plurality of layers, e.g., an input layer, an output layer, and at least one hidden layer, each having weights and/or parameters. As such, the artificial neural network extracts features based on the inputted data to identify patterns and, as a result, outputs predicted diagnoses associated with the inputted test images. The artificial neural network is trained to determine the diagnosis of the images captured by the imaging device 100 by comparing the predicted diagnoses with the actual diagnoses. The differences between the predicted diagnoses and the actual diagnoses provide a loss/cost function indicating a degree of error of the artificial neural network. Based on the loss/cost function, the weights and/or parameters of each layer of the artificial neural network are modified in order to reduce the loss/cost function. This process is repeated individually or with a subset of inputs for each of the inputs to repeatedly minimize the loss/cost function. This results in a trained module of the artificial neural network that may be utilized within the server 302 to determine the diagnoses, e.g., the presence of an abnormality, in each of the images captured by the imaging device 100.

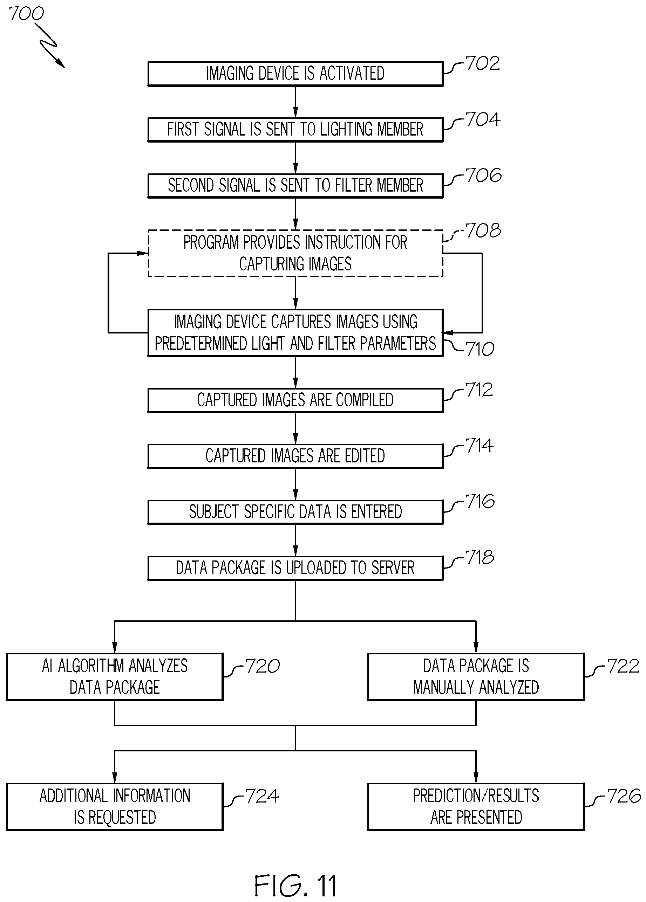

Referring now to FIG. 11, a process 700 is depicted for identifying the presence of an abnormality within a target area captured by an imaging device 100. The process 700 is discussed with reference to the imaging device 100 illustrated in FIGS. 1-6 and the abnormality detection system 300 illustrated in FIGS. 7-10. However, it should be appreciated that the process 700 may be used with additional or alternative devices and components not specifically described herein.

At block 702, the imaging device 100 is activated. Once activated, the imaging device 100 receives the default parameters for capturing an image of the target area of the subject. As noted above, the default parameters may be provided by the default parameters module 500 of the imaging device 100 and may be preset by an operator of the imaging device 100. The default parameters identify which combinations of lighting elements 230 of the lighting member 200 and filter elements 242 of the filter member 202 should be utilized to capture one or more images of the target area. In embodiments, as the imaging device 100 is activated and directed at a target area to be captured, the imaging device 100 may detect lighting conditions or initial characteristics of the target area such that the intelligent parameters module 502 may adjust the default parameters for the particular use of the imaging device 100. Specifically, the intelligent parameters module 502 may take into consideration the lighting conditions and initial characteristics of the target area to determine alternative and/or additional combinations of lighting elements 230 and filter elements 242 to be utilized when capturing the images of the target area.

Based on the determined combinations of lighting elements 230 and filter elements 242 to be utilized, at block 704, the controller 400 sends a first signal to the lighting member 200 to prepare one or more of the lighting elements 230 to be illuminated. The first signal may include instruction as to how the particular lighting elements 230 should be illuminated. For example, the instructions of the first signal may include specific tunable requirements to adjust a wavelength and/or a brightness of the light emitted from the lighting elements 230. At block 706, the controller 400 also sends a second signal to the filter member 202, in embodiments in which the filter member 202 is automatically rotatable, to cause the motor 237 to rotate the filter member 202 to position a specific one or more of the filter elements 242 in front of a corresponding one or more of the lighting elements 230.

At block 708, in embodiments, a program optionally provides instructions for capturing images using the imaging device 100. The program may be loaded onto, for example, the mobile device 306 in which initial subject data is inputted. For example, an operator may input subject data such as age, gender, nationality, history of preexisting illnesses or known skin abnormalities, and the like. The program processes this information to identify a set of instructions for capturing images of a target area of the subject. The set of instructions may include which lighting elements 230 should be illuminated, which filter elements 242 to be used, and the like. In other embodiments, the process 700 may proceed to block 710 without receiving instructions from the program, as discussed in block 708.

At block 710, the imaging device 100 is operated, such as by the operator directing the imaging device 100 at the target area and pressing the switch 126. In embodiments, upon pressing the switch 126, the imaging device 100 may capture a series of images with each image is captured utilizing a different combination of lighting elements 230 and filter elements 242. For example, a first image may be captured using a first combination of a lighting element 230 and a filter element 242, and a subsequent image may be captured using a second combination of a lighting element 230 and a filter element 242. As discussed herein, the filter member 202 may be rotated automatically with the use of a motor or gear such that a particular filter element 242 is positioned in front of a particular lighting element 230 to be illuminated. In another embodiment, the filter member 202 may be manually rotated by operating the adjustment member 236 accessible from the exterior of the imaging device 100 to position a particular filter element 242 in front of a particular lighting element 230 to be illuminated. Thereafter, the imaging device 100 may be instructed to capture another image upon the operator pressing the switch 126 using the new combination of lighting element 230 and filter element 242. Alternatively, the imaging device 100 may automatically capture subsequent images without additional operation of the switch 126 by the operator.

In embodiments, once the imaging device 100 captures the images, the program may process the captured images to identify whether additional images are required using different combinations of lighting elements 230 and filter elements 242. If so, the process 700 returns to block 708 in which the program provides instructions to capture additional images. Accordingly, the process 700 proceeds to block 710 to capture additional images in accordance with the instructions from the program. This process repeats until the program determines with a predetermined confidence level that a sufficient number of images have been captured to identify whether an abnormality is present.

The process 700 proceeds to block 712 in which the captured images are adjusted by the image adjustment module 504, as discussed herein. Particularly, the image adjustment module 504 will size and orient the captured images to highlight or focus on a target lesion in the target area on the subject's skin. Particularly, as discussed herein, the captured images may be cropped to remove extraneous imagery surrounding the target area and not necessary for processing. Thus, the total processing time may be reduced by removing the extraneous imagery and requiring processing of a smaller area captured by the imaging device 100. In addition, the captured images may be rotated to take into account movement or rotation of the imaging device 100 as the images were captured. Specifically, the image adjustment module 504 sets a plurality of target points in each captured image and orients each captured image such that the target points are in the same position relative to a vertical axis and a horizontal axis.

After the images are captured, the process 700 proceeds to block 714 in which the captured images are compiled by the image compilation module 506. In embodiments, the image compilation module 506 receives previously captured images taken by the imaging device 100 and/or previously captured images taken by a remote imaging device and received via the network interface hardware 404. Each of the previously captured images, in addition to the present images captured by the imaging device 100, are arranged chronologically so that the progression of the subject's skin can be assessed. In embodiments, the captured images may be viewed similar to a flip book in which a user may scroll between the captured images chronologically or allow the captured images to automatically transition in chronological order. In embodiments, the image adjustment module 504 and the image compilation module 506 may be configured to edit and compile the captured images simultaneously to ensure that each of the captured images in a single grouping of images, such as those taken within a particular range of time, have the same characteristics, such as being aligned in the same orientation, subjected to the same lighting constraints, and/or the like.

At block 716, in embodiments in which the subject data has not been inputted or entered for purposes of providing the intelligent parameters, the operator enters the subject data including age, gender, nationality, previous history of abnormalities, and the like. This subject data may be entered via the mobile device 306 or some other computing device communicatively coupled to the imaging device 100.

Thereafter, at block 718, a data package is created by the data package creation module 508. The data package includes the images captured by the imaging device 100 and the subject data. More particularly, the data package creation module 508 will create one or more merged image files each including a plurality of the captured images overlaid on top of one another. This provides the ability to analyze the images using multispectral imaging of the target area by simultaneously viewing various combinations of images captured with particular filter and lighting combinations. In embodiments, the data package may be created within the imaging device 100 itself or the captured images may be transmitted to the mobile device 306 or some other computing device which creates the data package. Accordingly, the data package is prepared and subsequently sent to the server 302 for processing the captured images to determine whether an abnormality is present within the target area of the captured images.

It should be appreciated that the data package may be analyzed using artificial intelligence or, alternatively, manually reviewed. In embodiments, the process proceeds to block 720 in which the server 302 analyzes the data package using artificial intelligence. Specifically, as discussed herein, the AI analysis module 602 of the server 302 is initially trained by processing test images stored within the image database 600 and associated diagnoses for each test image. Once trained, the AI analysis module 602 processes the captured images received from the imaging device 100. In embodiments, the AI analysis module 602 processes the image files each including a plurality of captured images captured using a different combinations of lighting elements 230 and filter elements 242 to identify characteristics of the target area that would not otherwise be identified by only a single lighting element 230 or a single filter element 242. Based on the analysis performed by the AI analysis module 602, the prediction module 604 of the server 302 establishes whether an abnormality has been detected. Alternatively, the prediction module 604 may identify a confidence level at which the AI analysis module 602 believes an abnormality has been detected. The confidence level may be determined based on any number of factors, such as, for example, the quality of the captured images, the number of the captured images, the similarity or closeness between the captured images and the test images stored in the image database 600, and/or the like. The confidence level may increase proportionally to an increased number of captured images being received and processed. Additionally, the similarity or closeness between the captured images and the test images may be determined by identifying similar characteristics in each, such as discolorations in the skin, sizes of potential lesions, and/or the like. As the number of similar characteristics between the captured images and the test images increases, the confidence level increases.

Alternatively or in addition thereto, at block 722, the data package may be manually analyzed by a technician. Manual analysis of the data package allows for a technician to confirm the diagnoses determined by the AI analysis module 602 to ensure that no misdiagnosis has occurred.

In embodiments, at block 724 the prediction module 604 or the manual reviewer may determine that additional images may need to be captured. Specifically, the prediction module 604 may determine that the confidence level is below a predetermined threshold and that additional images are required to be captured by the imaging device 100. Alternatively, if the confidence level is above a predetermined threshold, it is determined that no additional images need to be captured by the imaging device 100. Accordingly, at block 726, the prediction or results may be presented to the mobile device 306 and presented to the operator of the imaging device 100.

From the above, it is to be appreciated that defined herein are abnormality detection systems and methods for identifying a skin abnormality using multispectral imaging. Particularly, multispectral imaging is performed by operating an imaging device to capture images using a plurality of different combinations of lighting elements and filter elements. The filter elements are rotatable in front of the plurality of lighting elements and the captured images are compiled into a data package, which is transmitted to a server for processing and determining a presence of an abnormality.

Embodiments of the invention can be described with reference to the following numbered clauses, with preferred features laid out in the dependent clauses:
Clause 1. A method for identifying a skin abnormality comprising: using an imaging device comprising: a lighting member for directing light toward a target surface, the lighting member including a plurality of lighting elements; and a filter member positioned between the lighting member and the target surface, the filter member including a plurality of filter elements; capturing a plurality of images of the target surface, each of the plurality of images captured when illuminating a different one or more of the plurality of lighting elements; compiling the plurality of images into a data package; transmitting the data package to a server for processing the data package; and determining, at the server, a presence of an abnormality.
Clause 2. The method of clause 1, wherein the filter member is rotatably mounted within the imaging device, the filter member rotatable to selectively position one of the plurality of filter elements in front of any one of the plurality of lighting elements.
Clause 3. The method of clause 1 or clause 2, wherein the plurality of lighting elements are selected from the group consisting of an infrared lighting element and an ultraviolet lighting element.
Clause 4. The method of any of clauses 1-3, wherein the plurality of filters elements are selected from the group consisting of a white filter element, a green filter element, and a red filter element.
Clause 5. The method of any of clauses 1-4, wherein the data package including a plurality of image files, each of the plurality of image files including a different combination of the plurality of images overlaid on top of one another.
Clause 6. The method of any of clauses 1-5, further comprising inputting subject specific data into the data package, the subject specific data including demographic information relating to a subject.
Clause 7. The method of any of clauses 1-6, further comprising annotating at least one of the plurality of images to highlight a target lesion, the annotating including marking the at least one of the plurality of images to indicate one or more of a size and shape of the target lesion.
Clause 8. The method of any of clauses 1-7, further comprising assigning a time stamp to each of the plurality of images based on a time at which each of the plurality of images was captured.
Clause 9. The method of any of clauses 1-8, further comprising: retrieving one or more previously captured images; adding the one or more previously captured images to the data package; and chronologically arranging the one or more previously captured images among the plurality of images captured by the imaging device.
Clause 10. The method of any of clauses 1-9, further comprising: identifying one or more target points in each of the plurality of images; and editing the plurality of images such that the one or more target points in each of the plurality of images are positioned in the same location across the plurality of images, wherein editing the plurality of images includes at least one of rotating and cropping each of the plurality of images.
Clause 11. The method of any of clauses 1-10, wherein the determining the presence of an abnormality comprises using an artificial neural network to detect the presence of the abnormality and identify a type of abnormality, the artificial neural network is trained by analyzing a plurality of test images stored in an image database, determining a predicted diagnosis, comparing the predicted diagnosis to an actual diagnosis of the plurality of test images, adjusting an algorithm of the artificial neural network to minimize an error.
Clause 12. The method of any of clauses 1-11, further comprising: determining that one or more additional images are necessary to determine whether an abnormality is present in the plurality of images; and sending instruction to the imaging device to capture the one or more additional images of the subject, the instructions indicating a requirement to use one or more of the plurality of lighting elements and the plurality of filter elements.
Clause 13. An abnormality detection system comprising: an imaging device comprising: a lighting member for directing light toward a target surface, the lighting member including a plurality of lighting elements; a filter member positioned between the lighting member and the target surface, the filter member including a plurality of filter elements; and a controller configured to: capturing a plurality of images of the target surface, each of the plurality of images captured when illuminating a different one or more of the plurality of lighting elements; compile the plurality of images into a data package; and transmit the data package to a server for processing the data package; and a server communicatively coupled to the imaging device, the server comprising: a controller configured to: receive the data package including the plurality of images captured by the imaging device; and analyze the plurality of images of the data package to detect a presence of a skin abnormality.
Clause 14. The abnormality detection system of clause 13, the filter member is rotatably mounted within the imaging device, the filter member rotatable to selectively position one of the plurality of filter elements in front of any one of the plurality of lighting elements.
Clause 15. The abnormality detection system of clause 13 or clause 14, wherein the plurality of lighting elements are selected from the group consisting of an infrared lighting element and an ultraviolet lighting element.
Clause 16. The abnormality detection system of any of clauses 13-15, wherein the plurality of filter elements are selected from the group consisting of a white filter, a green filter, and a red filter.
Clause 17. The abnormality detection system of any of clauses 13-16, wherein the controller of the server is configured to: retrieve one or more previously captured images; add the one or more previously captured images to the data package; and chronologically arrange the one or more previously captured images among the plurality of images captured by the imaging device.
Clause 18. The abnormality detection system of any of clauses 13-17, wherein the controller of the server is configured to: identify one or more target points in each of the plurality of images; and edit the plurality of images such that the one or more target points in each of the plurality of images are positioned in the same location across the plurality of images by performing at least one of rotating and cropping each of the plurality of images.
Clause 19. The abnormality detection system of any of clauses 13-18, wherein the imaging device comprises a dermatoscope.
Clause 20. A dermatoscope comprising: a lighting member including a plurality of lighting elements; a filter member including a plurality of filter elements for filtering different frequencies of light emitted by the plurality of lighting elements; and a controller configured to: capture a plurality of images of a target surface using different combinations of the plurality of lighting elements and the plurality of filter elements by selectively illuminating a different one or more of the plurality of lighting elements for each of the plurality of images; compile the plurality of images into a data package; and transmit the data package to a server for processing the data package to identify a skin abnormality.
Clause 21. The dermatoscope of clause 20, wherein the filter member is rotatably mounted within the dermatoscope, the filter member rotatable to selectively position one of the plurality of filter elements in front of any one of the plurality of lighting elements.
Clause 22. The dermatoscope of clause 21, wherein the filter member is manually rotatable upon actuation of a thumb wheel accessible from an exterior of the dermatoscope.
Clause 23. The dermatoscope of clause 21, wherein the filter member is automatically rotatable in response to receiving an instruction indicating a requirement to capture one or more additional images using a particular lighting member and filter member combination.
Clause 24. The dermatoscope of any of clauses 20-23, wherein the plurality of lighting elements are selected from the group consisting of an infrared lighting element and an ultraviolet lighting element.
Clause 25. The dermatoscope of any of clauses 20-24, wherein the plurality of filters elements are selected from the group consisting of a white filter element, a green filter element, and a red filter element.
Clause 26. A method of manufacturing an imaging device comprising: using a housing including a first housing portion and a second housing portion defining an interior, a first aperture formed in the first housing portion and a second aperture formed in the second housing portion; positioning an eye piece within the first aperture of the first housing portion; mounting a lighting member within the interior of the housing between the first aperture and the second aperture, the lighting member including a plurality of lighting elements arranged in a spaced apart manner along an outer perimeter of the lighting member; rotatably mounting a filter member within the interior of the housing between the lighting member and the second aperture, the filter member including a plurality of filter elements arranged in a spaced apart manner along an outer perimeter of the filter member; and securing the first housing portion to the second housing portion.
Clause 27. The method of manufacturing an imaging device of clause 26, further comprising: rotatably mounting a thumb wheel partially within the housing and accessible from an exterior of the housing, wherein the filter member includes a plurality of teeth formed along an outer perimeter of the filter member, wherein the thumb wheel engages the plurality of teeth of the filter member such that rotation of the thumb wheel in one direction results in rotation of the filter member in an opposite direction.
Clause 28. The method of manufacturing an imaging device of clause 26 or clause 27, wherein the plurality of lighting elements are selected from the group consisting of an infrared lighting element and an ultraviolet lighting element.
Clause 29. The method of manufacturing an imaging device of any of clauses 26-28, wherein the plurality of filter elements are selected from the group consisting of a white filter, a green filter, and a red filter.
Clause 30. The method of manufacturing an imaging device of any of clauses 26-29, further comprising: positioning a hook formed at an upper end of the first housing portion within a receptacle formed at an upper end of the second housing portion; and positioning trim ring around a lower end of the first housing portion and a lower end of the second housing portion.

## Claims

1. A method for detecting a skin abnormality comprising:
using an imaging device comprising:
a lighting member for directing light toward a target surface, the lighting member including a plurality of lighting elements; and
a filter member positioned between the lighting member and the target surface, the filter member including a plurality of filter elements;
capturing a plurality of images of the target surface, each of the plurality of images captured when illuminating a different one or more of the plurality of lighting elements;
compiling the plurality of images into a data package;
transmitting the data package to a server for processing the data package; and
determining, at the server, a presence of an abnormality.

2. The method of claim 1, wherein the filter member is rotatably mounted within the imaging device, the filter member rotatable to selectively position one of the plurality of filter elements in front of any one of the plurality of lighting elements.

3. The method of any of claim 1 or claim 2, wherein:
the plurality of lighting elements are selected from the group consisting of an infrared lighting element and an ultraviolet lighting element; and
the plurality of filters elements are selected from the group consisting of a white filter element, a green filter element, and a red filter element.

4. The method of any of claims 1-3, wherein the data package including a plurality of image files, each of the plurality of image files including a different combination of the plurality of images overlaid on top of one another.

5. The method of any of claims 1-4, further comprising inputting subject specific data into the data package, the subject specific data including demographic information relating to a subject.

6. The method of any of claims 1-5, further comprising:
annotating at least one of the plurality of images to highlight a target lesion, the annotating including marking the at least one of the plurality of images to indicate one or more of a size and shape of the target lesion; and
assigning a time stamp to each of the plurality of images based on a time at which each of the plurality of images was captured.

7. The method of any of claims 1-6, further comprising:
retrieving one or more previously captured images;
adding the one or more previously captured images to the data package; and
chronologically arranging the one or more previously captured images among the plurality of images captured by the imaging device.

8. The method of any of claims 1-7, further comprising:
identifying one or more target points in each of the plurality of images; and
editing the plurality of images such that the one or more target points in each of the plurality of images are positioned in the same location across the plurality of images,
wherein editing the plurality of images includes at least one of rotating and cropping each of the plurality of images.

9. The method of any of claims 1-8, further comprising:
determining that one or more additional images are necessary to determine whether an abnormality is present in the plurality of images; and
sending instruction to the imaging device to capture the one or more additional images of the subject, the instructions indicating a requirement to use one or more of the plurality of lighting elements and the plurality of filter elements.

10. An imaging device comprising:
a lighting member for directing light toward a target surface, the lighting member including a plurality of lighting elements; and
a filter member positioned between the lighting member and the target surface, the filter member including a plurality of filter elements; and
a controller configured to:
capture a plurality of images of the target surface, each of the plurality of images captured when illuminating a different one or more of the plurality of lighting elements
compile the plurality of images into a data package; and
transmit the data package to a server for processing the data package.

11. An abnormality detection system comprising:
an imaging device according to claim 10; and
a server communicatively coupled to the imaging device, the server comprising:
a controller configured to:
receive the data package including the plurality of images captured by the imaging device; and
analyze the plurality of images of the data package to detect a presence of a skin abnormality.

12. The abnormality detection system of claim 11, the filter member is rotatably mounted within the imaging device, the filter member rotatable to selectively position one of the plurality of filter elements in front of any one of the plurality of lighting elements.

13. The abnormality detection system of claim 11 or claim 12, wherein:
the plurality of lighting elements are selected from the group consisting of an infrared lighting element and an ultraviolet lighting element; and
the plurality of filter elements are selected from the group consisting of a white filter, a green filter, and a red filter.

14. The abnormality detection system of any of claims 11-13, wherein the controller of the server is configured to:
retrieve one or more previously captured images;
add the one or more previously captured images to the data package; and
chronologically arrange the one or more previously captured images among the plurality of images captured by the imaging device.

15. The abnormality detection system of any of claims 11-14, wherein the controller of the server is configured to:
identify one or more target points in each of the plurality of images; and
edit the plurality of images such that the one or more target points in each of the plurality of images are positioned in the same location across the plurality of images by performing at least one of rotating and cropping each of the plurality of images.
